Europäisches Patentamt

European Patent Office

Office européen des brevets

(19)

(11) **EP 0 861 232 B1**

(12) **EUROPÄISCHE PATENTSCHRIFT**

(45) Veröffentlichungstag und Bekanntmachung des Hinweises auf die Patenterteilung:
**19.09.2001 Patentblatt 2001/38**

(51) Int Cl.[7]: **C07C 269/04**

(86) Internationale Anmeldenummer:
**PCT/EP96/04710**

(21) Anmeldenummer: **96937292.9**

(22) Anmeldetag: **30.10.1996**

(87) Internationale Veröffentlichungsnummer:
**WO 97/17323 (15.05.1997 Gazette 1997/21)**

(54) **VERFAHREN ZUR HERSTELLUNG VON ORGANISCHEN DIURETHANEN UND/ODER POLYURETHANEN UND IHRE VERWENDUNG ZUR HERSTELLUNG VON DI- UND/ODER POLYISOCYANATEN**

METHOD OF PRODUCING ORGANIC DIURETHANES AND/OR POLYURETHANES AND THEIR USE IN THE PRODUCTION OF DI- AND/OR POLYISOCYANATES

PROCEDE DE PRODUCTION DE DIURETHANES ET/OU DE POLYURETHANES ORGANIQUES ET LEUR UTILISATION POUR LA PRODUCTION DE DI- ET/OU DE POLY-ISOCYANATES

(84) Benannte Vertragsstaaten:
**AT BE CH DE DK ES FI FR GB GR IE IT LI LU MC NL PT SE**

(30) Priorität: **07.11.1995 DE 19541384**

(43) Veröffentlichungstag der Anmeldung:
**02.09.1998 Patentblatt 1998/36**

(73) Patentinhaber: **BASF AKTIENGESELLSCHAFT
67056 Ludwigshafen (DE)**

(72) Erfinder:
• **LAQUA, Gerhard
D-67117 Limburgerhof (DE)**
• **SCHONER, Ulrich
D-01987 Schwarzheide (DE)**
• **OTTERBACH, Andreas
D-67227 Frankenthal (DE)**
• **SCHWARZ, Hans, Volkmar
B-1410 Waterloo (BE)**

(56) Entgegenhaltungen:
**EP-A- 0 027 940        EP-A- 0 048 371**

Bemerkungen:
Die Akte enthält technische Angaben, die nach dem Eingang der Anmeldung eingereicht wurden und die nicht in dieser Patentschrift enthalten sind.

## Beschreibung

Gegenstand der Erfindung ist ein Verfahren zur Herstellung von aliphatischen oder cycloaliphatischen Diurethanen, durch Umsetzung der entsprechenden organischen Di- und/oder Polyamine (a) mit Harnstoff und/oder Carbamidsäurealkylestern (b) und Alkoholen (c) in Gegenwart von löslichen Zirkonverbindungen, wie z.B. Zirkonalkoholaten, Zirkonacetat oder Zirkonacetylacetonat, als Katalysator (d).

Organische Polyisocyanate, wie z.B. aromatische, aliphatische oder cycloaliphatische Polyisocyanate, sind wertvolle Ausgangsstoffe zur Herstellung von Polyisocyanat-polyadditionsprodukten, wie z.B. Polyurethan(PU)-Schaumstoffen, -Lacken, -Dispersionen, -Klebstoffen, Polyisocyanurat (PIR)-Schaumstoffen, thermoplastischen PU-Elastomeren (TPU) und kompakten oder zelligen PU- oder PU-Polyharnstoff-Elastomeren.

Die technischen Verfahren zur Herstellung organischer Polyisocyanate beruhen auf der Phosgenierung der entsprechenden organischen Polyamine zu Polycarbamidsäurechloriden und deren thermische Spaltung zu den Polyisocyanaten und Chlorwasserstoff und der thermischen Spaltung von monomeren Di- und/oder Polyurethanen in Di- und/oder Polyisocyanate und Alkohol.

Problematisch bei dem Phosgenierungsverfahren sind insbesondere der hohe Umsatz von Chlor über Phosgen und Carbamidsäurechlorid in Chlorwasserstoff, die Toxizität des Phosgens sowie die Korrosivität des Reaktionsgemisches, die Labilität der in der Regel eingesetzten Lösungsmittel und die Bildung halogenhaltiger Rückstände.

Es hat daher nicht an Versuchen gefehlt, organische Isocyanate, vorzugsweise aromatische und (cyclo)aliphatische Di- und/oder höherfunktionelle Polyisocyanate, nach einem phosgenfreien Verfahren herzustellen.

Nach Angaben der EP-A-28 338 (US-A-4 290 970) werden aromatische Di- und/oder Polyisocyanate hergestellt nach einem Zweistufenverfahren, wobei in der ersten Reaktionsstufe primäre aromatische Di- und/oder Polyamine mit Carbamidsäurealkylester in Abwesenheit oder Gegenwart von Katalysatoren und gegebenenfalls Harnstoff und Alkohol zu Aryl-di- und/oder -polyurethanen umgesetzt werden und das dabei gebildete Ammoniak gegebenenfalls abgetrennt wird und die erhaltenen Aryl-di- und/oder -polyurethane durch thermische Spaltung in der zweiten Reaktionsstufe in aromatische Di- und/ oder Polyisocyanate übergeführt werden.

Kontinuierliche mehrstufige Verfahren zur phosgenfreien Herstellung von organischen Polyisocyanaten sind ebenfalls bekannt.

Gegenstand der EP-A-0 355 443 (US-A-5 087 739) ist ein Kreislaufverfahren zur Herstellung von (cyclo) aliphatischen Diisocyanaten durch Umwandlung der entsprechenden Diamine in Diurethane und deren thermische Spaltung, das Ausbeuteminderungen reduziert, indem der Austrag der Urethanspaltungsstufe nach Umsetzung mit Alkohol in die Urethanisierungsstufe zurückgeführt wird. Die Abtrennung von nicht rückführbaren Nebenprodukten erfolgt durch eine destillative Auftrennung des Urethanisierungsaustrages, in der der unverwertbare Rückstand als Sumpfprodukt anfällt und alle leichter siedenden Komponenten, u.a. auch das Diurethan, über Kopf abgezogen werden.

Die EP-A-0 568 782 (US-A-5 360 931) beschreibt ein mehrstufiges Verfahren zur kontinuierlichen phosgenfreien Herstellung von (cyclo)aliphatischen Diisocyanaten, das die Umwandlung von (cyclo)aliphatischen Diaminen in einem Destillationsreaktor in die entsprechenden (Cyclo)alkylenbisharnstoffe, deren Umsetzung mit Alkohol in einem Druckdestillationsreaktor zu den (Cyclo)alkylenbiscarbamaten und die thermische Spaltung letzterer in einer kombinierten Spalt- und Rektifizierkolonne in die (Cyclo)alkylendiisocyanate und Alkohol in flüssiger Phase ohne Verwendung von Lösungsmitteln umfaßt.

Nach Angaben der EP-A-0 566 925 (US-A-5 386 053) werden in einem mehrstufigen Verfahren zur Herstellung von organischen Polyisocyanaten die organischen Polyamine mit Kohlensäurederivaten und Alkoholen in monomere Polyurethane übergeführt und diese thermisch gespalten, wobei in bestimmten Reaktionsstufen die hergestellten Polyisocyanate und unverwertbaren Rückstände abgetrennt und die wiederverwertbaren Nebenprodukte in Vorstufen zurückgeführt werden.

Die Wirtschaftlichkeit der kontinuierlichen phosgenfreien Verfahren zur Herstellung von organischen Polyisocyanaten wird maßgebend beeinflußt durch die Reinheit der zur thermischen Spaltung verwendeten organischen monomeren Di- und/oder höherwertigen Polyurethane und den teilweise aus Verunreinigungen und Nebenprodukten resultierenden unerwünschten Nebenreaktionen und der Tendenz der Reaktionsmischung zur Ausbildung von Belegungen, Verharzungen und Verstopfungen in Reaktoren und Aufarbeitungseinrichtungen.

In EP-A-48371 wird ein Verfahren zur Herstellung von Diurethanen durch Umsetzung von primären Aminen mit Dialkylcarbonaten in Gegenwart von Katalysatoren beschrieben. Als Katalysatoren werden neutrale oder basische anorganische oder organische Verbindungen des Bleis, Titans, Zinks oder Zirkons eingesetzt.

Organische monomere Di- und/oder höherwertige Polyurethane können durch Umsetzung von organischen Polyaminen mit Kohlensäurederivaten, vorzugsweise Harnstoff und/oder Carbamidsäurealkylestern, und Alkoholen in Abwesenheit oder Gegenwart von Katalysatoren hergestellt werden.

Nach Angaben der EP-B-0 018 586 (US-A-4 713 476 und US-A-4 851 565) können aliphatische und/oder cycloaliphatische Di- und/oder Polyurethane durch Umsetzung der entsprechenden Polyamine mit Harnstoff und Alkoholen in Gegenwart von Katalysatoren hergestellt werden. In analoger Weise können gemäß EP-B-0 019 109 (US-A-4 611 079) durch Verwendung von aromatischen Polyaminen aromatische Di- und/oder Polyurethane erhalten werden. Als geeignete Katalysatoren werden anorganische oder organische Verbindungen, die ein oder mehrere Kationen von Metallen der Gruppen IA, IB, IIA, IIB, IIIA, IIIB, IVA, IVB, VA, VB, VIB, VIIB und VIIIB des Periodensystems enthalten, z.B. Halogenide, Sulfate, Phosphate, Nitrate, Borate, Alkoholate, Phenolate, Sulfonate, Oxide, Oxidhydrate, Hydroxyde, Carboxylate, Chelate, Carbonate und Thiol- oder Dithiocarbamate, genannt. Arylmono-, -di- und/oder -polyurethane können nach Angaben der EP-B-0 018 538 (US-A-4 278 805 und US-A-4 375 000) durch Umsetzung entsprechender primärer aromatischer Mono- oder Polyamine mit Carbamidsäurealkylestern in Gegenwart der vorgenannten Katalysatoren hergestellt werden. N,O-disubstituierte Urethane können nach Angaben der EP-A-0 028 331 (US-A-4 480 110) durch Umsetzung von Mischungen aus substituierten Harnstoffen und N-unsubstituierten Urethanen und/oder Harnstoff und/oder Polyureten mit Alkoholen in Gegenwart von Veresterungskatalysatoren für Carbonsäuren hergestellt werden. Nach Angaben der EP-B-0 027 940 (CA-A-1 144 562) können Urethane durch Umsetzung von Harnstoff oder Polyureten mit primären Aminen und Alkoholen in Gegenwart von Verbindungen als Katalysatoren hergestellt werden, die einen katalytischen Einfluß auf die Veresterungsreaktion von Carbonsäuren mit Alkoholen ausüben und auch eine beschleunigende Wirkung auf die Urethanreaktion haben. Als geeignete Katalysatoren werden genannt: Unter den Reaktionsbedingungen inerte anorganische und organische Basen, Lewis-Säuren und Salze bzw. Komplexverbindungen, insbesondere Chelate von Übergangsmetallen. Unter einer Vielzahl verwendbarer Katalysatoren werden auch Koordinationsverbindungen von Eisen, Nickel, Kobalt, Zink, Mangan, Molybdän, Titan, Zirkon, Thorium, Hafnium und Vanadium mit β-Diketonen, z.B. Acetylaceton und β-Ketoester, beispielhaft genannt. Die beste katalytische Wirksamkeit zeigte Zinkoctoat mit einem Umsatz von 97 Mol-%, während Eisenacetylacetonat mit 90 Mol-% eine vergleichsweise, insbesondere für großtechnische Verfahren geringe Katalysatorwirkung ergab.

Um eine ausreichende Qualität der organischen Diurethane und/oder höherwertigen Polyurethane für eine thermische Spaltung zu organischen Polyisocyanaten zu gewährleisten, sind Herstellungsverfahren für Polyurethane erwünscht, die bei gleichzeitig hoher Raum-Zeit-Ausbeute eine hohe Selektivität, z.B. von 98 und mehr Molprozent Polyurethan, bezogen auf das eingesetzte organische Polyamin, gewährleisten.

Teilumgesetzte, harnstoffgruppenhaltige Zwischenprodukte stören in der Urethanspaltung zum Polyisocyanat erheblich und sie lassen sich nur schwer vom gebildeten Polyurethan abtrennen.

Die Aufgabe der vorliegenden Erfindung bestand darin, Di- und/ oder höherwertige Polyurethane in möglichst hohen Raum-Zeit-Ausbeuten mit hohen Selektivitäten herzustellen, um diese unter optimalen Reaktionsbedingungen, kostengünstig durch thermische Spaltung in Polyisocyanate überzuführen.

Diese Aufgabe konnte überraschenderweise gelöst werden durch eine spezielle Urethankatalyse.

Gegenstand der Erfindung ist somit ein Verfahren zur Herstellung von aliphatischen oder cycloaliphatischen Di- und/oder höherwertigen Polyurethanen, insbesondere von aliphatischen oder cycloaliphatischen Diurethanen, durch Umsetzung von

a) aliphatischen oder cycloaliphatischen Diaminen und/oder Polyaminen, mit
b) Harnstoff, Carbamidsäurealkylester oder Mischungen aus Harnstoff, Carbamidsäurealkylestern und/oder Dialkylcarbonaten
c) Alkoholen
    in Gegenwart eines
d) Katalysators,

das dadurch gekennzeichnet ist, daß man als Katalysator (d) in der Reaktionsmischung lösliche Zirkonverbindungen verwendet.

Da das für die Herstellung einer bestimmten Menge Di- und/oder Polyurethanen benötigte Reaktorvolumen direkt von der Raum-Zeit-Ausbeute der Umsetzung abhängig ist, kann durch die Verwendung der erfindungsgemäßen löslichen Zirkonverbindungen die Katalyse optimiert werden. Hieraus resultieren folgende ökonomische Vorteile: Es können die Kosten der Produktionsanlage minimiert werden, in bestehenden Produktionsanlagen kann die Polyurethanausbeute erhöht werden und/oder es können in bestehenden Produktionsanlagen definierte Polyurethanmengen mit maximaler Selektivität produziert werden. Das erfindungsgemäße Verfahren ermöglicht unter identischen Reaktionsbedingungen eine Steigerung der Raum-Zeit-Ausbeute um den Faktor 3 im Vergleich zu der unkatalysierten Umsetzung und um den Faktor 2 bei Einsatz des erfindungsgemäßen Zirkonacetats im Vergleich zu Aluminiumacetylacetonat gemäß EP-B-0 027 940. Mit dem erfindungsgemäßen Verfahren können in vorteilhafter Weise monomere aliphatishe und cycloaliphatische Di- und/oder Polyurethane, mit maximaler Selektivität in hohen Raum-Zeit-Ausbeuten hergestellt und dadurch die Herstellkosten minimiert werden.

Erfindungsgemäß finden als Katalysatoren Zirkonverbindungen Verwendung, die in der Reaktonsmischung, insbesondere unter den angewandten Reaktionsbedingungen löslich sind. Als lösliche Zirkonverbindungen in Betracht kommen z.B. Zirkonalkoholate von linearen oder verzweigten Alkoholen mit 1 bis 10 C-Atomen, vorzugsweise 1 bis 4 C-Atomen wie z.B. Zirkonmethanolat, -ethanolat, -n- und -isopropanolat, -n-butanolat, -sec-butanolat, -tert-butanolat, isomere Zirkonpentanolate, wie z.B. -tetrapentanolat, -2-methyl-2-butanolat, -hexanolat-, -2-ethylhexanolat, tetra-2-ethylhexanolat, -octanolat-, -decanolat und vorzugsweise Zirkon-n-butanolat, aminsubstituierte Zirkonverbindungen, wie z.B. Tetradiethylaminozirkon, Acetylacetonate, wie z.B. Zirkonacetylacetonat, Zirkon-tetra-2-ethylhexanoat, Zirkon (IV)trifluoracetylacetonat, Tetrakis(2,2,6,6-tetramethyl-3,5-heptandionato)zirkon, Zirkonhexafluoroacetylacetonat, Carboxylate des Zirkons, wie z.B. Zirkonacetat, Zirkonoxalat, Zirkonylpropionat, Zirkonbutyrat und Isomere hiervon, Zirkontetraacrylat, Zirkonmethacrylat, Zirkondimethacrylatdipropanolat, Zirkon(IV)dimethacrylat, Zirkonmethacryloxyethylacetonacetat-tri-n-propanolat und höhere Carboxylate, wie z.B. Zirkoncitrate, Zirkonlactat, Zirkonneodecanoat, Cyclopentadienylkomplexe des Zirkon, wie z.B. Zirkonocenediethoxid, Zirkonocendichlorid, Zirconocenchloridhydrid, Cyclopentadienylzirkoniumtrichlorid, Bis(pentamethylcyclopentadienyl)zirkondichlorid, Pentamethylcyclopentadienyl-zirkontrichlorid, Zirkonocenbis(trifluoromethansulfonat), Zirkonsalze anorganischer Säuren, wie z.B. Zirkoncarbonat, Zirkoniumhydroxyd, Zirkonnitrat, Zirkonsulfat, Zirkonsulfid, Zirkonphosphat, Zirkonpyrophosphat, Zirkonylammoniumcarbonat, Zirkonhalogenide, wie z.B. Zirkon(IV)fluorid, Zirkon(IV)chlorid, Zirkoniumtetrabromid, Zirkontetraiodid, Zirkonylchlorid, Zirkonylperchlorat, Natriumhexafluorozirkonat, Zirkonate, wie z.B. Aluminiumzirkonat, Ammoniumhexafluorozirkonat, Bismutzirkonat, Bleizirkonat, Cadmiumzirkonat, Caesiumzirkonat, Calciumzirkonat, Cerzirkonat, Cobaltzirkonat, Kaliumhexafluorozirkonat, Kaliumpentafluorozirkonat, Lithiumzirkonat, Magnesiumzirkonat, Manganzirkonat, Natriumzirkonat, Rubidiumzirkonat, Strontiumzirkonat, Zinkzirkonat und andere anorganische Zirkonverbindungen, wie z.B. Zirkonaluminid, Zirkonborid, Zirkonhydrid, Zirkoncarbid, Zirkonmolybdat, Zirkonnitrid, Zirkonselenid, Zirkontelurid und Zirkonwolframat.

Sehr gut bewährt haben sich und daher werden vorzugsweise verwendet: Zirkonacetat, Zirkonacetylacetonat und insbesondere Zirkon-n-butanolat.

Die Katalysatoren können in beliebigen Mengen eingesetzt werden. Aus wirtschaftlichen Gründen werden vorteilhafterweise möglichst geringe Katalysatormengen verwendet. In Abhängigkeit von ihrer katalytischen Wirksamkeit, die experimentell einfach gemessen werden kann, werden die Zirkonverbindungen zweckmäßigerweise in einer Menge von 0,00001 bis 1 Molprozent, vorzugsweise von 0,0001 bis 0,1 Molprozent und insbesondere von 0,001 bis 0,05 Molprozent verwendet.

Die organischen monomeren aliphatischen und/oder cycloaliphatischen Di- und/oder Polyurethane können in Gegenwart der erfindungsgemäß geeigneten Katalysatoren nach den bekannten Verfahren durch Umsetzung von Polyaminen mit Kohlensäurederivaten, insbesondere Harnstoff, und Alkoholen unter Abtrennung des entstehenden Ammoniak hergestellt werden.

Rein formal betrachtet kann das erfindungsgemäße Verfahren durch folgende Gleichung bilanziert werden:

$$R\text{-}(NH_2)_n + n\ H_2NCONH_2 + n\ R'OH$$

$$\Delta \downarrow Katalysator$$

$$R\text{-}(NCOOR')_n + 2n\ NH_3$$

a) Als Amine der Formel $R\text{-}(NH_2)_n$, in der R einen aliphatischen oder cycloaliphatischen Rest und n eine ganze Zahl bedeuten, deren Wert der Valenzzahl von R entspricht und wenigstens 2, vorzugsweise 2 bis 5 und insbesondere 2, beträgt, kommen beispielweise in Betracht: aliphatische Polyamine mit 2 bis 19 C-Atomen, vorzugsweise 3 bis 13 C-Atomen und insbesondere 4 bis 6 C-Atomen; diese können eine geradkettige oder verzweigte Struktur besitzen und als Brückenglieder Heteroatome, wie z.B. Sauerstoff oder Schwefel, oder zweiwertige heterocyclische Reste oder Arylen-, vorzugsweise Phenylenreste, gebunden enthalten, wie z.B. Ethylen-, 1,3- und 1,2-Propylen-, 2,2-Dimethyl-1,3-propylen-, 1,4-Butylen-, 2-Ethyl-1,4-butylen-, 2-Ethyl-2-butyl-1,4-butylen-, 1,5-Pentamethylen-, 2-Methyl-1,5-pentamethylen-, 2-Ethyl-2-butyl-1,5-pentamethylen-, 1,6-Hexamethylen-, 2,2,4-Trimethyl-1,6-hexamethylen-, 1,8-Octamethylen, 1,10-Decylen, 1,12-Dodecylen-, 1,4-Hexahydroxylylen- und cycloaliphatische Polyamine mit 5 bis 12 C-Atomen, vorzugsweise 6 bis 12 C-Atomen, wie z.B. 1,2-, 1,3- und 1,4-Cyclohexan-diamin, 2,4- und 2,6-Hexahydrotoluylen-diamin sowie die entsprechende Isomerengemische, 4,4'-, 2,4'- und 2,2'-Diamino-dicyclohexylmethan sowie die entsprechenden Isomerengemische und 3-Aminomethyl-3,5,5-trimethylcyclohexylamin Als Polyamine, vorzugsweise Diamine finden vorzugsweise Verwendung 1,6-Hexamethylen-diamin und 3-Aminomethyl-3,5,5-trimethylcyclohexylamin.

b) Als Kohlensäurederivat wird vorzugsweise Harnstoff (b) verwendet. Da Harnstoff mit Alkohol unter den angewandten Reaktionsbedingungen der Urethanisierung Carbamidsäurealkylester bildet, kann anstelle von Harnstoff oder gemeinsam mit Harnstoff auch ein Carbamidsäurealkylester als Aufbaukomponente (b) verwendet werden. In einer Vorstufe kann z.B. aus Harnstoff und Alkohol, der zweckmäßigerweise in einer überschüssigen Menge verwendet wird, ein Carbamidsäurealkylester hergestellt und dieser z.B. in Form einer alkoholischen Lösung mit den Polyaminen in Gegenwart der löslichen Zirkonverbindungen als Katalysator zur Reaktion gebracht werden. In analoger Weise können auch bei der Urethanisierung als Nebenprodukt gebildete oder separat hergestellte Dialkylcarbonate eingesetzt werden, so daß anstelle von Harnstoff Mischungen aus Harnstoff, Carbamidsäurealkylestern und/oder Dialkylcarbonat verwendet werden können.

Geeignete Carbamidsäurealkylester besitzen die Formel $H_2N$-COOR, in der R einen gegebenenfalls substituierten aliphatischen, cycloaliphatischen oder aromatisch-aliphatischen Rest bedeutet. In Betracht kommen beispielsweise Carbamidsäurealkylester auf Basis von primären aliphatischen Monoalkoholen mit 1 bis 20 Kohlenstoffatomen, vorzugsweise 1 bis 10 Kohlenstoffatomen, wie z.B. Carbamidsäure-methylester, -ethylester, -propylester, -n-butylester, -isobutylester, -2- und -3-methylbutylester, -neopentylester, -pentylester, -2-methyl-pentylester, -n-hexylester, -2-ethylhexylester, -heptylester, -n-octylester, -n-nonylester, -n-decylester und -n-dodecylester, -2-phenylpropylester und -benzylester und auf Basis von sekundären aliphatischen und cycloaliphatischen Monoalkoholen mit 3 bis 15 Kohlenstoffatomen, vorzugsweise 3 bis 6 Kohlenstoffatomen, wie z.B. Carbamidsäure-isopropylester, -sek.-butylester, -sek.-isoamylester, -cyclopentylester, -cyclohexyl-ester, tert.-butyl-cyclohexylester und -bicyclo-(2,2,1)-heptylester. Vorzugsweise verwendet werden Carbamidsäure-methylester, -ethylester, -propylester, -butylester, -isobutylester, -2- und -3-methylbutylester, -pentylester, -hexylester, -2-ethylhexylester, -heptylester, -octylester und -cyclohexylester.

Sofern die Urethanisierung mit Harnstoff in Gegenwart von Dialkylcarbonaten oder vorzugsweise Carbamidsäurealkylestern durchgeführt wird, so können die Dialkylcarbonate z.B. in einer Menge von 0,1 bis 30 Mol-%, vorzugsweise 1 bis 10 Mol-%, oder die Carbamidsäurealkylester z.B. in einer Menge von 1 bis 20 Mol-%, vorzugsweise von 5 bis 15 Mol-%, bezogen auf das Polyamin, vorzugsweise Diamin verwendet werden. Insbesondere verwendet werden jedoch Mischungen aus Dialkylcarbonaten und Carbamidsäurealkylestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Die Herstellung der monomeren organischen Polyurethane, vorzugsweise Diurethane kann ferner in Gegenwart anderer Kohlensäurederivate, z.B. von Polyureten, substituierten Harnstoffen, unsubstituierten und substituierten Polyharnstoffen, Oligoharnstoff-polyurethanen, hochsiedenden Oligomeren und anderen bei der thermischen Spaltung der Di- und/oder Polyurethanen zu Di- und/oder Polyisocyanaten entstehenden Nebenprodukten durchgeführt werden. Diese Kohlensäurederivate können z.B. bei der thermischen Spaltung der Polyurethane gebildet, isoliert, gegebenenfalls zwischengelagert oder in getrennten Verfahren hergestellt und gegebenenfalls zusammen mit dem Harnstoff (b) und/oder Alkohol (c) in die Polyurethanherstellung eingebracht werden. Bei kontinuierlichen Verfahren zur Herstellung von organischen Polyisocyanaten durch thermische Spaltung von Polyurethanen können derartige Kohlensäurederivate aus dem Spaltreaktor in die Urethanisierung zurückgeführt werden.

c) Als Alkohole (c) können für das erfindungsgemäße Verfahren beliebige, gegebenenfalls substituierte primäre oder sekundäre aliphatische Alkohole oder aromatisch-aliphatische Alkohole sowie Mischungen davon verwendet werden. Da die Di- und/ oder Polyurethane insbesondere zur Herstellung von Polyisocyanaten verwendet werden, wird man jedoch vorzugsweise solche auswählen, deren Siedepunkte genügend weit vom Siedepunkt des durch die thermische Spaltung erhaltenen Polyisocyanates, vorzugsweise Diisocyanates, entfernt liegen, so daß eine möglichst quantitative Trennung der Spaltprodukte Polyisocyanat, vorzugsweise Diisocyanat und Alkohol möglich ist.

Aus diesen Gründen finden daher vorzugsweise primäre aliphatische Monoalkohole mit 1 bis 20 C-Atomen, vorzugsweise 1 bis 10 C-Atomen, wie Methanol, Ethanol, Propanol, n-Butanol, Isobutanol, 2- und 3-Methylbutanol, Neopentylalkohol, Pentanol, 2-Methyl-pentanol, n-Hexanol, 2-Ethylhexanol, n-Heptanol, n-Octanol, n-Nonanol, n-Decanol, n-Dodecanol, 2-Phenylpropanol und Benzylalkohol, sekundäre aliphatische und cycloaliphatische Monoalkohole mit 3 bis 15 C-Atomen, vorzugsweise 3 bis 6 C-Atomen, wie Isopropanol, sec-Butanol, sec-Isoamylalkohol, Cyclopentanol, Cyclohexanol, 2,3- oder 4-Methyl-cyclohexanol und 4-tert.-Butyl-cyclohexanol oder Gemische der genannten Alkohole, insbesondere aber n-Butanol und/oder isoButanol Verwendung.

Zur Herstellung der Di- und/oder Polyurethane nach dem erfindungsgemäßen Verfahren können die aliphatischen und/oder cycloaliphatischen primären organischen Di- und/oder Polyamine (a), vorzugsweise aliphatischen oder cycloaliphatischen Diamine, mit Harnstoff (b) und Alkohol (c) in solchen Mengen zur Reaktion gebracht werden, daß das Verhältnis von $NH_2$-Gruppen der Amine zu Harnstoff (b) sowie gegebenenfalls Carbamidsäurealkylester und/ oder Dialkylcarbonat zu Hydroxylgruppen der Alkohole (c) 1:0,7 bis 5:1 bis 50, vorzugsweise 1:0,9 bis 2:1 bis 10 und insbesondere 1:1,0 bis 1,3:1,5 bis 5 beträgt. Wird die Umsetzung in Gegenwart von Dialkylcarbonaten und/oder vorzugsweise Carbamidsäurealkylestern durchgeführt, so kann anstelle von 1 Mol Harnstoff 1 Mol Carbamidsäurealkylester oder Dialkylcarbonat eingesetzt werden. Die Reaktion in Gegenwart der löslichen Zirkonverbindungen als Katalysator wird üblicherweise bei einer Temperatur im Bereich von 160° bis 300°C, vorzugsweise von 180° bis 250°C und insbesondere von 185° bis 240°C und unter einem Druck, der in Abhängigkeit von dem verwendeten Alkohol zwischen 0,1 bis 60 bar, vorzugsweise 1 bis 40 bar liegt, durchgeführt. Für diese Reaktionsbedingungen ergeben sich Reaktionszeiten von z.B. 0,5 bis 48, vorzugsweise 1 bis 3 Stunden.

Die Umsetzung wird vorteilhafterweise in Gegenwart von überschüssigem Alkohol als Lösungs- und Reaktionsmittel durchgeführt. Hierbei hat es sich als vorteilhaft erwiesen, das entstehende Ammoniak sofort aus der Reaktionsmischung, beispielsweise durch Destillation, abzutrennen. Eine hierfür verwendete Vorrichtung, z.B. eine Destillationskolonne kann bei Temperaturen von 60 bis 150°C, vorzugsweise von 65 bis 120°C betrieben werden, so daß eine Belegung durch Ammoniumcarbaminat, welches in minimalen Mengen aus Ammoniak und Kohlendioxid durch Zersetzung von Harnstoff gebildet wird, vermieden werden kann.

Die Di- und/oder Polyurethanherstellung kann chargenweise oder kontinuierlich durchgeführt werden. Bei der kontinuierlichen Verfahrensweise hat es sich als vorteilhaft erwiesen, den Alkohol, die Dialkylcarbonate, sofern solche gebildet wurden oder in der Reaktionsmischung vorliegen, oder Carbamidsäurealkylester oder Mischungen aus mindestens zwei dieser Komponenten nach beendeter Urethanisierung aus der Reaktionsmischung abzutrennen und vorzugsweise an den Reaktionsbeginn zurückzuführen. Zur Abtrennung der Komponenten wird die Reaktionsmischung vorteilhafterweise vom Druckniveau des Reaktionsbeginns auf einen Druck im Bereich von 1 bis 500 mbar, vorzugsweise von 10 bis 100 mbar entspannt. Man erhält hierbei gasförmige Brüden, die die überwiegende Alkoholmenge sowie 0 bis 30 Gew.-%, vorzugsweise 1 bis 10 Gew.-% Dialkylcarbonat und/oder 1 bis 50 Gew.-%, vorzugsweise 1 bis 20 Gew.-% Carbamidsäurealkylester enthalten, und einen flüssigen Austrag, der im wesentlichen aus dem monomeren Polyurethan, vorzugsweise Diurethan, besteht und gegebenenfalls Oligoharnstoff-polyurethanen und hochsiedende Oligomere enthält.

Die erhaltenen Brüden können in nachfolgenden zweckmäßigerweise destillativen Reinigungsstufen, vorzugsweise durch Rektifikation, getrennt und die hierbei isolierten Wertprodukte Alkohol und Carbamidsäurealkylester, einzeln oder als Mischung, vorzugsweise an dem Reaktionsbeginn zur Bildung der monomeren Polyurethane zurückgeführt werden.

Der flüssige Austrag kann destillativ getrennt werden, z.B. mit einer üblichen Destillationsanlage, vorzugsweise einem Dünnschichtverdampfer, bei einer Temperatur von z.B. 170° bis 240°C, vorzugsweise von 180° bis 230°C und unter einem Druck von 0,01 bis 5 mbar, vorzugsweise 0,1 bis 2 mbar, in ein Wertprodukt, das die Polyurethane, vorzugsweise Diurethane, und die leichter siedenden Nebenprodukte enthält, und einen nicht verwertbaren Rückstand, der verworfen wird.

Das Wertprodukt kann direkt durch thermische Spaltung in Di- und/ oder Polyisocyanate übergeführt werden oder es können die leichter siedenden Nebenprodukte abgetrennt werden, z.B. durch Destillation, und die erhaltenen Di- und/oder Polyurethane können durch eine Destillation oder durch Umkristallisation aus zweckmäßigerweise anderen Lösungsmitteln zusätzlich gereinigt werden.

Die nach dem erfindungsgemäßen Verfahren hergestellten organischen Polyurethane, vorzugsweise Diurethane, finden vorzugsweise Verwendung zur Herstellung von organischen Polyisocyanaten, die ihrerseits zur Herstellung von Polyisocyanat-polyadditionsprodukten verwendet werden.

Beispiele

Vergleichsbeispiel I

In einem 1,5 l Rührautoklaven, ausgestattet mit einer Druckhaltung und einem Rückflußkühler, der mit auf 90°C temperiertem Wasser betrieben wurde, wurden

    104,4 g (0,9 mol) Hexamethylen-diamin (HDA),
    135 g (2,25 mol) Harnstoff und
    532,8 g (7,2 mol) n-Butanol

bei 210°C unter Rückflußkühlung und unter einem Druck von 9 bar zur Reaktion gebracht. Der entstehende Ammoniak wurde kontinuierlich aus der Reaktionsmischung abgetrennt.

Durch eine stündliche Entnahme von Meßproben aus der Reaktionsmischung und ihre Analyse durch Flüssigkeitschromatographie wurde der Reaktionsverlauf messend verfolgt.

Nach einer Reaktionszeit von 6 Stunden war das Reaktionssystem noch nicht bezüglich aller entstehenden Nebenprodukte stationär. Der Hexamethylen-diurethan (HDU)-Gehalt im Austrag (660 g) betrug 40 Gew.-%. Dies ergab eine Raum-Zeit-Ausbeute von weniger als 53 [g/1•h].

Vergleichsbeispiel II

Man verfuhr analog den Angaben des Vergleichsbeispiels I, führte die Umsetzung jedoch in Gegenwart von 14,6 mg Aluminiumacetylacetonat durch (0,005 mol-%, bezogen auf HDA).

Das Reaktionssystem war nach 4 Stunden stationär, wobei der HDU-Gehalt im Austrag (661 g) 40 Gew.-% betrug. Dies ergab eine Raum-Zeit-Ausbeute von 80 [g/l•h].

Vergleichsbeispiel III

Man verfuhr analog den Angaben des Vergleichsbeispiels I, führte die Umsetzung jedoch in Gegenwart von 16 mg Eisenacetylacetonat durch (0,005 mol-%, bezogen auf HDA).

Das Reaktionssystem war nach 4 Stunden stationär, wobei der HDU-Gehalt im Austrag (660 g) 41 Gew.-% betrug. Dies ergab eine Raum-Zeit-Ausbeute von 82 [g/1•h].

Beispiel 1

Man verfuhr analog den Angaben des Vergleichsbeispiels I, führte die Umsetzung jedoch in Gegenwart von 22 mg Zirkonacetylacetonat durch (0,005 mol-%, bezogen auf HDA).

Das Reaktionssystem war nach 2 Stunden stationär, wobei der HDU-Gehalt im Austrag (663 g) 41 Gew.-% betrug. Dies ergab eine Raum-Zeit-Ausbeute von 164 [g/l•h].

Beispiel 2

Man verfuhr analog den Angaben des Vergleichsbeispiels I, führte die Umsetzung jedoch in Gegenwart von 10 mg Zirkonacetat ($Zr(C_2H_3O_2)_{1,4}(OH)_{2,6}$) durch (0,005 mol-%, bezogen auf HDA).

Das Reaktionssystem war nach 2 Stunden stationär, wobei der HDU-Gehalt im Austrag (662 g) 42 Gew.-% betrug. Dies ergab eine Raum-Zeit-Ausbeute von 168 [g/1•h].

Beispiel 3

Man verfuhr analog den Angaben des Vergleichsbeispiels I, führte die Umsetzung jedoch in Gegenwart von 21,6 mg einer 80-gew.-%igen Zirkonbutanolatlösung in n-Butanol durch (0,005 mol-%, bezogen auf HDA).

Das Reaktionssystem war nach 2 Stunden stationär, wobei der HDU-Gehalt im Austrag (662 g) 43 Gew.-% betrug. Dies ergab eine Raum-Zeit-Ausbeute von 172 [g/1•h].

Vergleichsbeispiel IV

In einem 1,5 1 Rührautoklaven, ausgestattet mit einer Druckhaltung und einem Rückflußkühler, der mit auf 90°C temperiertem Wasser betrieben wurde, wurden

170 g (1 mol) Isophoron-diamin (IPDA),
150 g (2,50 mol) Harnstoff und
592 g (8,0 mol) n-Butanol

bei 210°C unter Rückflußkühlung und unter einem Druck von 8 bar zur Reaktion gebracht. Der entstehende Ammoniak wurde kontinuierlich aus der Reaktionsmischung abgetrennt.

Durch eine stündliche Entnahme von Meßproben aus der Reaktionsmischung und ihre Analyse durch Flüssigkeitschromatographie wurde der Reaktionsverlauf messend verfolgt.

Nach einer Reaktionszeit von 6 Stunden war das Reaktionssystem noch nicht bezüglich aller entstehenden Nebenprodukte stationär. Die Raum-Zeit-Ausbeute betrug weniger als 60 [g/l•h].

Beispiel 4

Man verfuhr analog den Angaben des Vergleichsbeispiels IV, führte die Umsetzung jedoch in Gegenwart von 24,4 mg Zirkonacetylacetonat durch (0,005 mol-%, bezogen auf IPDA).

Das Reaktionssystem war nach 2 Stunden stationär, woraus sich eine Raum-Zeit-Ausbeute von 170 [g/l•h] Isophoron-diurethan (IPDU) ergab.

Vergleichsbeispiel V

In einem 1,5 1 Rührautoklaven, ausgestattet mit einer Druckhaltung und einem Rückflußkühler, der mit auf 90°C temperiertem Wasser betrieben wurde, wurden

104,4 g (0,9 mol) HDA,
263 g (2,25 mol) Carbamidsäure-n-butylester und

366 g (4,95 mol) n-Butanol

in Gegenwart von 14,6 mg Aluminiumacetylacetonat (0,005 mol-%, bezogen auf HDA) bei 210°C unter Rückflußkühlung und unter einem Druck von 9 bar zur Reaktion gebracht. Der entstehende Ammoniak wurde kontinuierlich aus der Reaktionsmischung abgetrennt.

Durch eine stündliche Entnahme von Meßproben aus der Reaktionsmischung und ihre Analyse durch Flüssigkeitschromatographie wurde der Reaktionsverlauf messend verfolgt.

Nach einer Reaktionszeit von 6 Stunden war das Reaktionssystem noch nicht bezüglich aller entstehenden Nebenprodukte stationär. Der HDU-Gehalt im Austrag (665 g) betrug 40,4 Gew.-%. Dies ergab eine Raum-Zeit-Ausbeute von weniger als 54 [g/l•h].

Beispiel 5

Man verfuhr analog den Angaben des Vergleichsbeispiels V, führte die Umsetzung jedoch in Gegenwart von 21,9 mg Zirkonacetylacetonat durch (0,005 mol-%, bezogen auf HDA).

Das Reaktionssystem war nach 4 Stunden stationär, wobei der HDU-Gehalt im Austrag (663 g) 42,6 Gew.-% betrug. Dies ergab eine Raum-Zeit-Ausbeute von 83 [g/l•h].

Vergleichsbeispiel VI

In einer aus 4 Rührkesseln bestehenden Rührkesselkaskade wurden kontinuierlich

600 g/h HDA,
700 g/h Harnstoff und
2200 g/h n-Butanol

unter folgenden Bedingungen zur Reaktion gebracht

| 1. Rührkessel | Volumen | 25 l |
| | Druck | 11 bar |
| | Temperatur | 218°C |
| | | |
| Zulauf | 600 g/h | HDA |
| | 700 g/h | Harnstoff |
| | 2200 g/h | n-Butanol |
| Kondensat | 14 l/h | n-Butanol |
| | | |
| 2. Rührkessel | Volumen | 12,5 l |
| | Druck: | 11 bar |
| | Temperatur | 222°C |
| | | |
| 3. Rührkessel | Volumen | 12,5 l |
| | Druck | 11 bar |
| | Temperatur | 227°C |
| | | |
| 4. Rührkessel | Volumen | 12,5 l |
| | Druck | 11 bar |
| | Temperatur | 230°C |

Der HDU-Gehalt im Kaskadenaustrag (3100 g/h) betrug 51,6 Gew.-%, woraus sich eine Raum-Zeit-Ausbeute von 26 [g/l•h] ergab. Die HDU-Ausbeute, bezogen auf eingesetztes HDA, betrug 97,9 %.

Vergleichsbeispiel VII

In einer aus 4 Rührkesseln bestehenden Rührkesselkaskade wurden kontinuierlich

1000 g/h HDA,
1200 g/h Harnstoff und
3700 g/h n-Butanol

unter folgenden Bedingungen zur Reaktion gebracht

| 1. Rührkessel | Volumen | 25 l |
|---|---|---|
| | Druck | 11 bar |
| | Temperatur | 218°C |
| Zulauf | 1000 g/h | HDA |
| | 1200 g/h | Harnstoff |
| | 3700 g/h | n-Butanol |
| Kondensat | 20 l/h | n-Butanol |
| Katalysator | 418 mg Aluminiumacetylacetonat (0,015 mol-%, bezogen auf HDA) | |
| 2. Rührkessel | Volumen | 12,5 l |
| | Druck | 11 bar |
| | Temperatur | 222°C |
| 3. Rührkessel | Volumen | 12,5 l |
| | Druck | 11 bar |
| | Temperatur | 227°C |
| 4. Rührkessel | Volumen | 12,5 l |
| | Druck | 11 bar |
| | Temperatur | 230°C |

Der HDU-Gehalt im Kaskadenaustrag (5200 g/h) betrug 49,4 Gew.-%, woraus sich eine Raum-Zeit-Ausbeute von 41 [g/1·h] ergab. Die HDU-Ausbeute, bezogen auf eingesetztes HDA, betrug 94,3 %.

Beispiel 6

In einer aus 4 Rührkesseln bestehenden Rührkesselkaskade wurden kontinuierlich

1300 g/h HDA,
1550 g/h Harnstoff und
4800 g/h n-Butanol

unter folgenden Bedingungen zur Reaktion gebracht

| 1. Rührkessel | Volumen | 25 l |
|---|---|---|
| | Druck | 11 bar |
| | Temperatur | 218°C |
| Zulauf | 1300 g/h | HDA |
| | 1550 g/h | Harnstoff |
| | 4800 g/h | n-Butanol |
| Kondensat | 26 l/h | n-Butanol |
| Katalysator | 127 mg Zirkonacetat (0,005 mol-%, bezogen auf HDA) | |
| 2. Rührkessel | Volumen | 12,5 l |
| | Druck | 11 bar |
| | Temperatur | 222°C |

(fortgesetzt)

| 3. Rührkessel | Volumen | 12,5 1=l |
| --- | --- | --- |
| | Druck | 11 bar |
| | Temperatur | 227°C |
| | | |
| 4. Rührkessel | Volumen | 12,5 l |
| | Druck | 11 bar |
| | Temperatur | 230°C |

Der HDU-Gehalt im Kaskadenaustrag (6800 g/h) betrug 51,0 Gew.-%, woraus sich eine Raum-Zeit-Ausbeute von 56 [g/l•h] ergab. Die HDU-Ausbeute, bezogen auf eingesetztes HDA, betrug 98,0 %.

Die Vergleichsbeispiele VI und VII sowie Beispiel 6 zeigen, daß in einer gegebenen, kontinuierlich betriebenen Rührkesselkaskade die für die nachfolgende Spaltungen erforderlichen hohen HDU-Aus-beuten mit den erfindungsgemäß löslichen Zirkonverbindungen als Katalysator in der höchsten Raum-Zeit-Ausbeute erzielt werden. Im Vergleich zur unkatalysierten Umsetzung (Vergleichsbeispiel VI), kann mit dem erfindungsgemäß verwendbaren Zirkonacetat als Katalysator (Beispiel 6) bei vorgegebener einzuhaltender HDU-Qualität dieselbe Rührkesselkaskade mit mehr als der doppelten Last betrieben werden.

**Patentansprüche**

1.  Verfahren zur Herstellung von aliphatischen und/oder cycloaliphatischen organischen Diurethanen und/oder Polyurethanen durch Umsetzung von

    a) aliphatischen und/oder cycloaliphatischen organischen Di- und/oder Polyaminen mit
    b) Harnstoff, Carbamidsäurealkylestern oder Mischungen aus Harnstoff, Carbamidsäurealkylestern und/oder Dialkylcarbonaten
    c) Alkohol
        in Gegenwart eines
    d) Katalysators,

    **dadurch gekennzeichnet, daß** man als Katalysator in der Reaktionsmischung lösliche Zirkonverbindungen verwendet.

2.  Verfahren nach Anspruch 1, **dadurch gekennzeichnet, daß** man als lösliche Zirkonverbindungen Zirkon-n-butanolat, Zirkonacetat oder Zirkonacetylacetonat verwendet.

3.  Verfahren nach Anspruch 1 oder 2, **dadurch gekennzeichnet, daß** man die löslichen Zirkonverbindungen in einer Menge von 0,00001 bis 1 mol%, bezogen auf die Di- und/oder Polyamine verwendet.

4.  Verfahren nach einem der Ansprüche 1 bis 3, **dadurch gekennzeichnet, daß** man als organische Diamine Hexandiamin-1,6 oder 3-Aminomethyl-3,5,5-trimethylcyclohexylamin verwendet.

5.  Verfahren nach einem der Ansprüche 1 bis 4, **dadurch gekennzeichnet, daß** man die Di- und/oder Polymamine (a), Harnstoff (b) und Alkohole (c) in solchen Mengen umsetzt, daß das Verhältnis von $NH_2$-Gruppen der Di- und/ oder Polyamine (a) zu Harnstoff (b) zu Hydroxylgruppen der Alkohole (c) 1:0,7 bis 5:1 bis 50 beträgt.

6.  Verfahren nach einem der Ansprüche 1 bis 5, **dadurch gekennzeichnet, daß** man die Umsetzung bei Temperaturen im Bereich von 160°C bis 300°C und einem Druck von 0,1 bis 60 bar durchführt.

7.  Verfahren nach einem der Ansprüche 1 bis 6, **dadurch gekennzeichnet, daß** man die Umsetzung in Gegenwart von Polyureten, substituierten Harnstoffen, substituierten und unsubstituierten Polyharnstoffen, Oligoharnstoff- polyurethanen, hochsiedenden Oligomeren und anderen bei der thermischen Spaltung der Di- und/oder Polyurethane zu Di- und/oder Polyisocyanaten entstehenden Nebenprodukten durchführt.

**Claims**

1. A process for preparing aliphatic and/or cycloaliphatic organic diurethanes and/or polyurethanes by reacting

   a) aliphatic and/or cycloaliphatic organic di- and/or polyamines with
   b) urea, alkyl carbamates or mixtures of urea, alkyl carbamates and/or dialkyl carbonates
   c) alcohol
      in the presence of a
   d) catalyst,

   wherein the catalyst used comprises zirconium compounds soluble in the reaction mixture.

2. A process as claimed in claim 1, wherein the soluble zirconium compounds used are zirconium-n-butoxide, zirconium acetate or zirconium acetylacetonate.

3. A process as claimed in claim 1 or 2, wherein the soluble zirconium compounds are used in an amount of 0.00001 to 1 mol%, based on the diamines and/or polyamines.

4. A process as claimed in any of claims 1 to 3, wherein the organic diamines used are 1,6-hexanediamine or 3-aminomethyl-3,5,5-trimethylcyclohexylamine.

5. A process as claimed in any of claims 1 to 4, wherein the diamines and/or polyamines (a), urea (b) and alcohols (c) are reacted in such amounts that the ratio of $NH_2$ groups of the diamines and/or polyamines (a) to urea (b) to hydroxyl groups of the alcohols (c) is 1:0.7-5:1-50.

6. A process as claimed in any of claims 1 to 5, wherein the reaction is carried out at from 160°C to 300°C and a pressure of from 0.1 to 60 bar.

7. A process as claimed in any of claims 1 to 6, wherein the reaction is carried out in the presence of polyurets, substituted ureas, substituted and unsubstituted polyureas, oligourea-polyurethanes, high-boiling oligomers and other by-products formed in the thermal dissociation of the diurethanes and/or polyurethanes to give diisocyanates and/or polyisocyanates.

**Revendications**

1. Procédé de préparation de diuréthannes et/ou de polyuréthannes organiques aliphatiques et/ou cycloaliphatiques par réaction de

   a) diamines et/ou polyamines organiques aliphatiques et/ou cycloaliphatiques avec
   b) de l'urée, des alkylesters de l'acide carbamique (carbamates d'alkyle) ou des mélanges d'urée, de carbamates d'alkyle et/ou de carbonates de dialkyle
   c) un alcool
   en présence de
   d) un catalyseur,

   **caractérisé en ce que** l'on utilise comme catalyseur des composés de zirconium solubles dans le mélange réactionnel.

2. Procédé selon la revendication 1, **caractérisé en ce que** l'on utilise comme composé soluble de zirconium du n-butanolate de zirconium, de l'acétate de zirconium ou de l'acétylacétonate de zirconium.

3. Procédé selon la revendication 1 ou 2, **caractérisé en ce que** l'on met en oeuvre les composés solubles de zirconium à raison de 0,00001 à 1% molaire, par rapport aux diamines et/ou polyamines.

4. Procédé selon l'une des revendications 1 à 3, **caractérisé en ce que** l'on utilise comme diamines organiques de l'hexadiamine-1,6 ou de la 3-aminométhyl-3,5,5-triméthylcyclohexylamine.

5. Procédé selon l'une des revendications 1 à 4, **caractérisé en ce que** l'on fait réagir les diamines et/ou polyamines (a), l'urée (b) et les alcools (c) en quantités telles que la proportion de groupes $NH_2$ des diamines ou polyamines (a) à l'urée (b) et aux groupes hydroxyle des alcools (c) soit de 1:0,7 à 5:1 à 50.

6. Procédé selon l'une des revendications 1 à 5, **caractérisé en ce que** la réaction est entreprise à des températures de l'ordre de 160°C à 300°C et sous une pression de 0,1 à 60 bar.

7. Procédé selon l'une des revendications 1 à 6, **caractérisé en ce que** l'on entreprend la réaction en présence de polyurets, d'urées substituées, de polyurées substituées et non substituées, d'oligo-urée-polyuréthannes, d'oligomères à haut point d'ébullition et d'autres sous-produits qui se forment lors de la décomposition thermique des di- et/ou polyuréthannes en di- et/ou polyisocyanates.